(19) Europäisches Patentamt
European Patent Office
Office européen des brevets

(11) Veröffentlichungsnummer: **0 224 612**
**A1**

# (12) EUROPÄISCHE PATENTANMELDUNG

(21) Anmeldenummer: 85115487.2

(51) Int. Cl.⁴: **C07D 233/64**

(22) Anmeldetag: 05.12.85

(43) Veröffentlichungstag der Anmeldung:
**10.06.87 Patentblatt 87/24**

(84) Benannte Vertragsstaaten:
**AT BE CH DE FR GB IT LI LU NL SE**

(71) Anmelder: **HEUMANN PHARMA GMBH & CO**
**18-28 Heideloffstrasse**
**D-8500 Nürnberg(DE)**

(72) Erfinder: **Schickaneder, Helmut, Dr. Dipl.-Chem.**
**Moosäcker 25**
**D-8501 Eckental(DE)**
Erfinder: **Kisielowski, Lothar, Dr. Dipl.-Chem.**
**Meuschelstrasse 1**
**D-8500 Nürnberg(DE)**
Erfinder: **Ahrens, Kurt Henning, Dr.**
**Praterstrasse 9**
**D-8500 Nürnberg(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

## (54) Verfahren zur Herstellung von N-Cyano-N'-methyl-N"[2-[[(5-methyl-1H-imidazol-4-yl)methyl]thio]ethyl]guanidin.

(57) Es wird ein neues zweistufiges Verfahren zur Herstellung von N-Cyano-N'-methyl-N"-[2-[[(5-methyl-1H-imidazol-4-yl)methyl]thio]ethyl]guanidin und dessen physiologisch annehmbarer Salze beschrieben, das über ein neues Zwischenprodukt, nämlich N-Cyano-N'-[2-[[(5-methyl-1H-imidazol-4-yl)methyl]thio]ethyl]-O-phenylisoharnstoff, abläuft. Dieses Verfahren kann zweistufig oder als Eintopfverfahren geführt werden und ermöglicht die billigere Herstellung von Cimetidin unter Verwendung von billigeren und reaktiveren Stoffen, als es nach dem Stand der Technik der Fall ist.

EP 0 224 612 A1

## Verfahren zur Herstellung von N-Cyano-N'-methyl-N"-[2-[[(5-methyl-1H-imidazol-4-yl)methyl]thio]ethyl]-guanidin

Die Erfindung bezieht sich auf ein neues Verfahren zur Herstellung von N-Cyano-N'-methyl-N"[2-[[(5-methyl-1H-imidazol-4-yl)methyl]thio] ethyl]guanidin und dessen physiologisch annehmbarer Salze. Diese als Cimetidin (INN) bezeichnete Verbindung ist bereits aus den Deutschen Offenlegungsschriften DOS 23 30 131 und DOS 2 344 779 bekannt.

Die vorliegende Erfindung bezieht sich außerdem auf die neue Verbindung N-Cyano-N'-[2-[[(5-methyl-1H-imidazol-4-yl)methyl]thio]ethyl] -O-phenylisoharnstoff und ihre Salze.

Cimetidin wird als Antihistaminikum für $H_2$-Rezeptoren eingesetzt. Seine therapeutische Bedeutung liegt in der Behandlung von Magengeschwüren durch Hemmung der histaminstimulierten Sekretion von Magensäure.

Zur Herstellung von Cimetidin sind verschiedene Verfahren bekannt. Von industrieller Bedeutung ist bisher jedoch hauptsächlich die in der Deutschen Patentschrift 23 44 779 beschriebene Umsetzung von 2-[-[(5-Methyl-1H-imidazol-4-yl)methyl]thio]ethylamin mit Dimethylcyanodithioimidocarbonat und Reaktion des daraus resultierenden Produkts mit Methylamin.

Der Erfindung liegt die Aufgabe zugrunde, die bekannten Verfahren zur Herstellung von Cimetidin zu verbessern und insbesondere ein neues Verfahren zu dessen Herstellung zur Verfügung zu stellen, welches unter Verwendung billiger und reaktiverer Stoffe durchgeführt werden kann.

Diese Aufgabe wird erfindungsgemäß dadurch gelöst, daß man

a) 2-[[(5-Methyl-1H-imidazol-4-yl)methyl]thio]ethylamin der Formel II

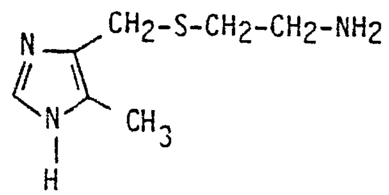

(II)

mit N-Cyanodiphenylimidocarbonat der Formel III

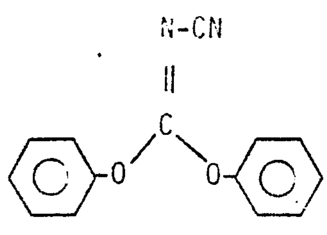

(III)

zu N-Cyano-N'-[2-[[(5-methyl-1H-imidazol-4-yl)methyl]thio]ethyl]-O-phenylisoharnstoff der tautomeren Formeln IV

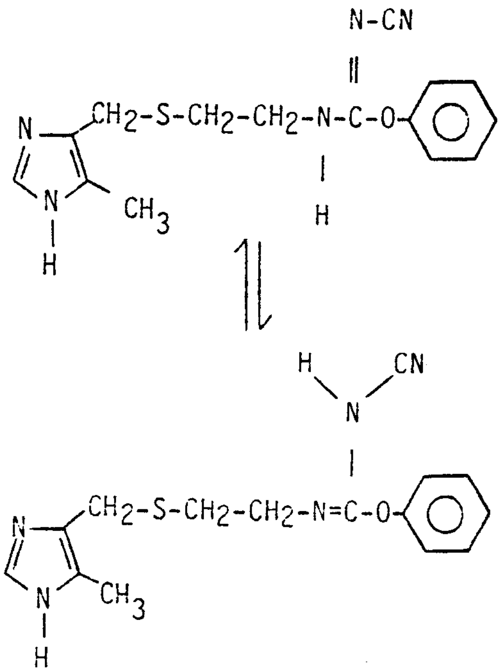

(IV)

umsetzt:

b) die erhaltene Verbindung der tautomeren Formeln IV mit Methylamin zu N-Cyano-N'-methyl-N''-[2-[[(5-methyl-1H-imidazol-4-yl)methyl]thio]ethyl]guanidin der Formel I umsetzt; und

c) gegebenenfalls die in Stufe (b) erhaltene Base in an sich bekannter Weise in ihr physiologisch annehmbares Salz, vorzugsweise in das Hydrochlorid, umwandelt.

Die Erfindung gestattet es, Cimetidin unter milden Reaktionsbedingungen -falls gewünscht, in einem Eintopfverfahren -durch einfache Gewinnungs-und Reinigungsverfahren in verbesserter Ausbeute und hoher Reinheit darzustellen.

Die Vorteile dieses neuen Verfahrens liegen in der Verwendung des billigen und reaktiveren N-Cyanodiphenylimidocarbonats anstelle des teuren 5,5-Dimethyl-N-cyanodithioimidocarbonats. Dies stellt einerseits einen wirtschaftlichen Vorteil dar. Vor allem muß auf die ökologischen Vorteile dieses Verfahrens hingewiesen werden. Im Gegensatz zur oben erwähnten bisherigen Herstellungsmethode ensteht bei dem neuen Verfahren kein toxisches und übelriechendes Methylmercaptan.

Im folgenden wird das erfindungsgemäße Verfahren anhand der einzelnen Stufe näher beschrieben:

I. Stufe (a):

In der ersten Stufe setzt man das aus der DE-OS 22 11 454 bekannte 2-[[(5-Methyl-1H-imidazol-4-yl)-methyl]thio]ethylamin der Formel II

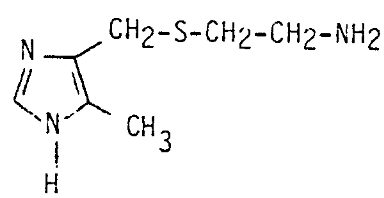

(II)

das zum Beispiel aus seinem Bishydrochlorid durch Behandlung mit Triethylamin oder Natriummethanolat freigesetzet werden kann, mit äquimolaren Mengen N-Cyanodiphenylimidocarbonat der Formel III

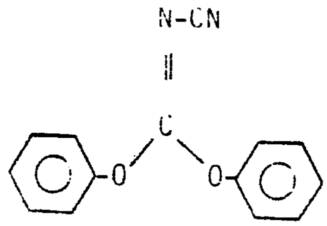

(III)

[R.L. Webb, C.S. Labaw, J. Heterocyclic. Chem. 19, 1205 (1982)] um.

Gemäß einer bevorzugten Ausführungsform wird das Amin der Formel II direkt im Reaktionsgemisch auf seinem Bishydrochlorid der Formel V

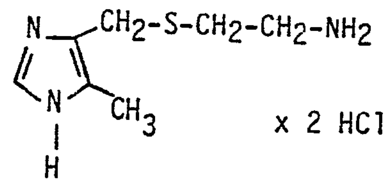

(V)

und einer Hilfsbase, wie zum Beispiel Triethylamin oder Natriummethanolat, in situ erzeugt.

Diese Stufe läßt sich in einem Alkanol, Keton oder Ether durchführen, wobei die Reaktionstemperatur zwischen -10 und +30°C gehalten wird. Beispiele für geeignete Alkanole sind insbesondere Ethanol und Methanol, Isopropanol und n-Butanol, während Beispiele für geeignete Ketone insbesondere Aceton und Methylethylketon sind. Als Ether werden Tetrahydrofuran (THF) und Dioxan bevorzugt.

So kann man beispielsweise zur quantitativen Herstellung von N-Cyano-N'-[2-[[(5-methyl-1H-imidazol-4-yl)methyl]thio]ethyl]-O-phenylisoharnstoff der tautomeren Formeln IV

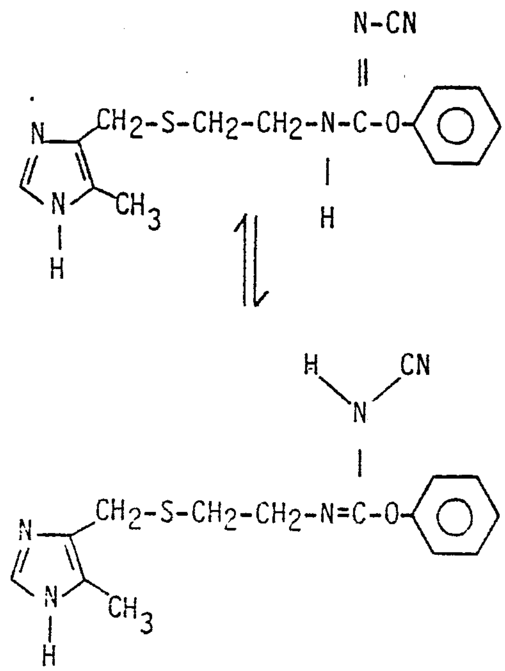

(IV)

so vorgehen, daß man bei -5 bis +20°C die zweifache äquimolare Menge Triethylamin zur Suspension von 2-[[(5-Methyl-1H-imidazol-4-yl) methyl]thio]ethylamin-Bishydrochlorid und N-Cyanodiphenylimidocarbonat in Isopropanol gibt und weitere 2 Stunden bei Raumtemperatur rührt. Wie oben angegeben, kann N-Cyano-N'-[2-[[(5-methyl-1H-imidazol-4-yl)methyl]thio]ethyl]-O-phenylisoharnstoff je nach Lösungsmittelbedingung in den tautomeren Formeln IV vorliegen; deshalb umfaßt das erfindungsgemäße Verfahren auch die Herstellung der zweiten isomeren Form. Die Isolierung des auf diese Weise hergestellten N-Cyano-N'-[2-[[(5-methyl-1H-imidazol-4-yl)methyl]thio]ethyl]-O-phenylisoharnstoffs erfolgt nach allgemeinen Verfahrensweisen, beispielsweise Umkristallisation etc.

II. Stufe (b):

In der zweiten Stufe setzt man den nach Stufe a) erhaltenen N-Cyano-N'-[2-[[(5-methyl-1H-imidazol-4-yl)methyl]thio]ethyl]-O-phenylisoharnstoff der tautomeren Formel IV mit überschüssigem Methylamin quantitativ zu N-Cyano-N'-methyl-N"-[2-[[(5-methyl-1H-imidazol-4-yl)methyl] thio]ethyl]guanidin der Formel I um. Die Umsetzung führt man in einem Alkanol, Keton oder Ether durch, wobei Reaktionstemperaturen zwischen 20 und 117°C bevorzugt werden. Beispiele für geeignete Alkanole sind insbesondere Ethanol, Methanol, Isopropanol und n-Butanol, während Beispiele für geeignete Ketone insbesondere Aceton und Methylethylketon sind. Als Ether werden Tetrahydrofuran (THF) und Dioxan bevorzugt. n-Butanol oder Isopropanol werden als Lösungsmittel bevorzugt. Das Methylamin als Reaktionspartner wird vorzugsweise im Überschuß, beispielsweise im Molverhältnis von 3:1 bis 5:1 zu dem Reaktionspartner N-Cyano-N'-[2[[(5-methy-1H-imidazol-4-yl)methyl]thio]ethyl]-O-phenylisoharnstoff, eingesetzt. Dabei kann das Methylamin gasförmig oder in Lösung des bei der Reaktion verwendeten Lösungsmittels oder vorzugsweise in wäßriger Lösung zugegeben werden. Die Reaktionszeit beträgt je nach Temperaturwahl 0,5 bis 2 Stunden. Wie oben erwähnt, lassen sich die Reaktionsstufen a) und b) gewünschtenfalls in einem Eintopfverfahren durchführen, wobei naturgemäß am Ende der Stufe a) die Isolierung des Zwischenprodukts N-Cyano-N'-[2-[[(5-methyl-1H-imidazol-4-yl)methyl] thio] ethyl] -O-phenylisoharnstoff der tautomeren Formel IV entfällt.

Das in Stufe b) erhaltene N-Cyano-N'-methyl-N"-[2-[[(5-methyl-1H-imidazol-4-yl)methyl]thio]ethyl]-guanidin kann -falls gewünscht -in an sich bekannter Weise in ein physiologisch annehmbares Salz umgewandelt werden. Dieses Salz kann sich zum Beispiel von einer Mineralsäure, wie Salzsäure, Salpeter- oder Schwefelsäure, oder von einer organischen Säure herleiten.

Die nach dem erfindungsgemäßen Verfahren erhaltene Verbindung kann zur Verabreichung in gleicher Weise formuliert werden, wie es für Cimetidin und dessen Salze, insbesondere das Hydrochlorid, bereits bekannt ist.

Die als Zwischenprodukt bei dem erfindungsgemäßen Verfahren entstehende Verbindung N-Cyano-N'-[2-[[(5-methyl-1H-imidazol-4-yl) methyl]thio]ethyl]-O-phenylisoharnstoff (Formel IV) ist neu und läßt sich auch als Säureadditionsprodukt herstellen. Salze lassen sich z.B. mit einer Mineralsäure, wie Salzsäure, Brom-und Jodwasserstoffsäure, Phosphorsäure, Salpetersäure oder Schwefelsäure, herstellen.

Die Verbindung N-Cyano-N'-[2-[[(5-methyl-1H-imidazol-4-yl)methyl] thio]ethyl]-O-phenylisoharnstoff der tautomeren Formeln IN

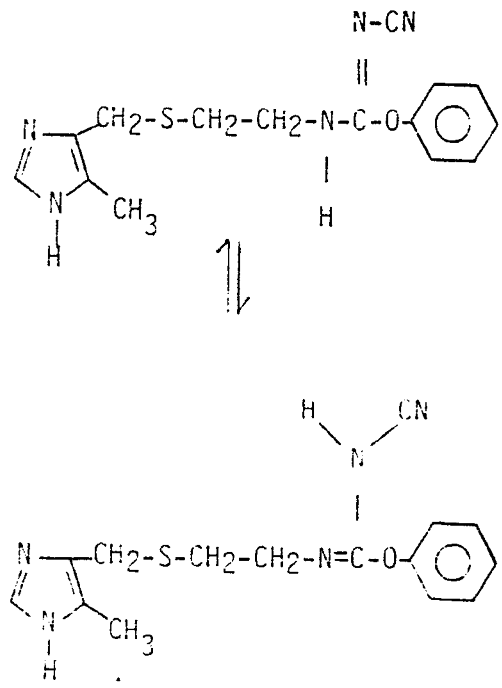

(IV)

und ihre Salze sind daher ebenfalls Gegenstand der vorliegenden Erfindung.

Die Erfindung wird in den folgenden Beispielen erläutert.

Beispiel 1

Herstellung von N-Cyano-N'-[2-[[(5-methyl-1H-imidazol-4-yl]methyl] thio]ethyl-O-phenylisoharnstoff

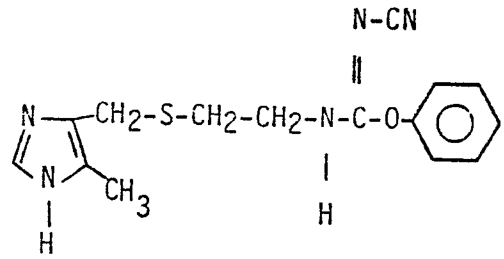

Zu einer Suspension von 23.8 g (100 mmol) N-Cyanodiphenylimidocarbonat und 24.4 g (100 mmol) 2-[-[(5-Methyl-1H-imidazol-4-yl)methyl]thio]ethylamin-Bishydrochlorid in 120 ml Isopropanol tropft man unter Rühren bei 0 -10° C 28 ml (200 mmol) Triethylamin. Man rührt eine weitere Stunde bei Raumtemperatur, saugt die ausgefallenen Salze ab, zieht das Lösungsmittel im Vakuum ab und nimmt den Rückstand in 150 ml heißem THF auf. Nach Abfiltrieren von unlöslichen Salzen kristallisiert beim Abkühlen N-Cyano-N'-[2-[[-(5-methyl -1H-imidazol-4-yl)methyl]thio]ethyl]-O-phenylisoharnstoff.

Ausbeute: 29.3 g (93 % d.Th.)
Schmelzpunkt: 134,8 -135,4°C
Rf-Wert: 0.6 (Dichlormethan/MeOH 80:20)
$^1$H-NMR-Daten: ($d_6$-DMSO, TMS als interner Standard) $\delta$ = 2.15 (s) (Imidazol-$CH_3$) 3 H,
2.75 (m) (S-$CH_2$) 2 H,
3.5 (m) (N-$CH_2$ 2 H
3.71 (s) (Imidazol-$CH_2$-S) 2 H,
7,37 (m) (Aromaten-H) 6 H,
8.73 (breit) (NH) 2 H ppm.

Beispiel 2

Herstellung von N-Cyano-N'-methyl-N''-[2-[[(5-methyl-1H-imidazol-4-yl)methyl]thio]ethyl]guanidin

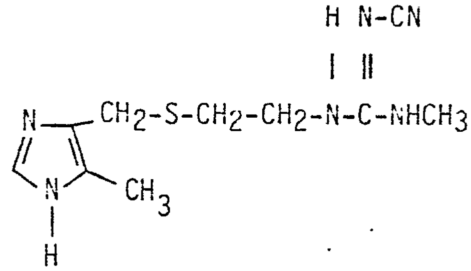

Durch eine Suspension von 15.8 g (50 mmol) des in Beispiel 1 erhaltenen N-Cyano-N'-[2-[[(5-methyl-1H-imidazol-4-yl)methyl] thio] ethyl] -O-phenylisoharnstoffs in 80 ml Isopropanol leitet man einen -schwachen Methylamin-Strom, bis 5 g Methylamin absorbiert sind. Man rührt 2 Stunden, saugt danach ab und kristallisiert aus Isopropanol um.

Ausbeute: 11,4 g (91 % d.Th.)
Schmelzpunkt: 141 -143° C
Rf: 0.48 (Methylenchlorid/Methanol 80:20)

$^1$H-NMR-Daten: ($d_6$-DMSO, TMS als interner Standard) $\delta$ = 2.16 (s) (Imidazol-$CH_3$) 3 H,
2.60 (m) (S-$CH_2$) 2 H,
2.73 (d) (N-$CH_3$) 3 H,
3.33 (m) (N-$CH_2$) 2 H,
3.66 (s) (Imidazol-$CH_2$-S) 2 H,
7.23 (m) (NH) 2 H,
7.55 (s) (Imidazol-H) 1 H,
10.13 (breit) (NH) 1 H ppm.

Beispiel 3

Herstellung von N-Cyano-N'-methyl-N''-[2-[[(5-methyl-1H-imidazol-4-yl)methyl]thio]ethyl]guanidin (Eintopfverfahren).

Zu einer Suspension von 23.8 g (100 mmol) N-Cyanoimidodiphenylcarbonat und 24.4 g 2-[[(5-Methyl-1H-imidazol-4-yl)methyl]thio]ethylamin-Bishydrochlorid in 120 ml n-Butanol tropft man unter Rühren bei 0 - 10° C 28 ml (200 mmol) Triethylamin. Man rührt eine weitere Stunde bei Raumtemperatur und gibt dann in einer Portion 30 ml wäßrige Methylaminlösung (40 %) zu. Nach 2stündigem Rühren versetzt man mit 100 ml Wasser, trennt die Phasen, zieht das n-Butanol im Vakuum ab und digeriert den Rückstand mit 200 ml tert.-Butylmethylether. Das Produkt wird abgesaugt und aus Isopropanol umkristallisiert.

Ausbeute: 21.2 g Cimetidin (84 %)
Schmelzpunkt: 141 -143° C
Rf-Wert: 0.48 (Methylenchlorid/MeOH 80:20)

## Ansprüche

1. Verfahren zur Herstellung von N-Cyano-N'-methyl-N''-[2-[[(5-methyl-1H-imidazol-4-yl)methyl]thio]-ethyl]guanidin der Formel I

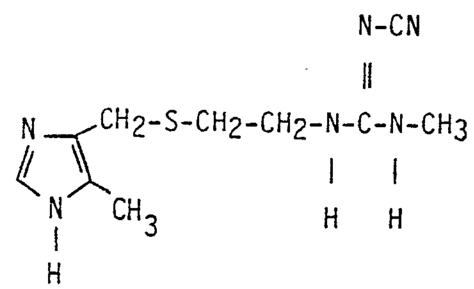

(I)

und dessen physiologisch annehmbarer Salze, dadurch **gekennzeichnet**, daß man

a) 2-[[(5-Methyl-1H-imidazol-4-yl)methyl]thio]ethylamin der Formel II

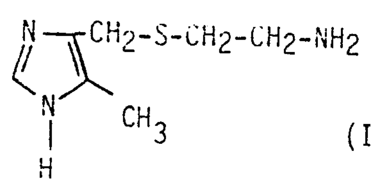

(II)

mit N-Cyanodiphenylimidocarbonat der Formel III

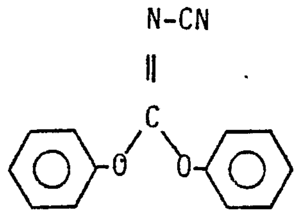

(III)

zu N-Cyano-N'-[2[[(5-methyl-1H-imidazol-4-yl)methyl]thio]ethyl]-O-phenylisoharnstoff der tautomeren Formeln IV

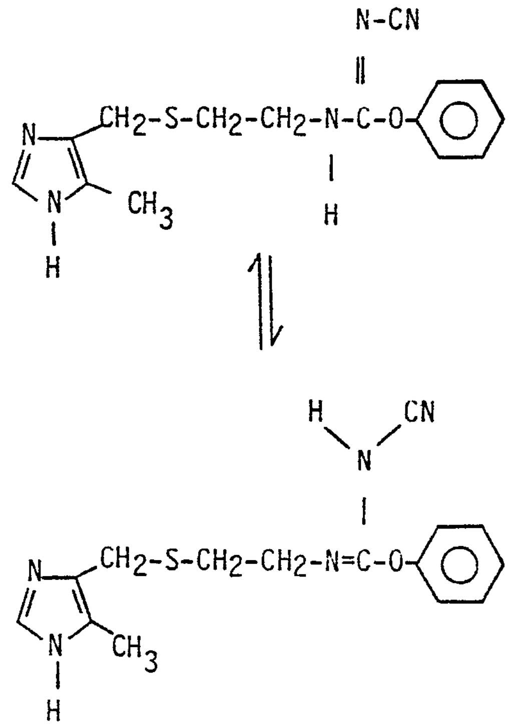

(IV)

umsetzt;

b) die erhaltene Verbindung der tautomeren Formeln IV mit Methylamin zu N-Cyano-N'methyl-N''-[2-[[(5-methyl-1H-imidazol -4-yl)methyl]thio] ethyl]guanidin (Formel I) umsetzt; und

c) gegebenenfalls die in Stufe b) erhaltene Base in an sich bekannter Weise in ihr physiologisch annehmbares Salz, vorzugsweise das Hydrochlorid, umwandelt.

2. Verfahren nach Anspruch 1, dadurch **gekennzeichnet,** daß man die Stufen a) und b) in einem Keton bzw. einem Alkanol, bevorzugt Isopropanol oder n-Butanol, oder einem Ether, wie zum Beispiel THF, als Lösungsmittel durchführt.

3. Verfahren nach Anspruch 1 oder 2, dadurch **gekennzeichnet,** daß man das in Stufe a) verwendete 2-[[(5-Methyl-1H-imidazol-4-yl)methyl]thio]ethylamin der Formel II in situ aus seinem Bishydrochlorid mit einer Hilfsbase, vorzugsweise Triethylamin oder Natriummethanolat, herstellt.

4. Verfahren nach den Ansprüchen 1 bis 3, dadurch **gekennzeichnet,** daß man die Stufen a) und b) bei einer Temperatur von -10°C bis Rückflußtemperatur des Lösungsmittels, bevorzugt bei -10 bis +30°C, durchführt.

5. Verfahren nach den Ansprüchen 1 bis 4, dadurch **gekennzeichnet,** daß man die Stufen a) und b) in einem "Eintopfverfahren" durchführt.

6. Verfahren nach den Ansprüchen 1 bis 3 oder 5, dadurch **gekennzeichnet,** daß man in Stufe b) das Methylamin gasförmig oder in Form einer 40%igen wäßrigen Lösung verwendet.

7. N-Cyano-N'-[2-[[(5-methyl-1H-imidazol-4-yl)methyl]thio]ethyl]-O-phenylisoharnstoff der tautomeren Formeln IV

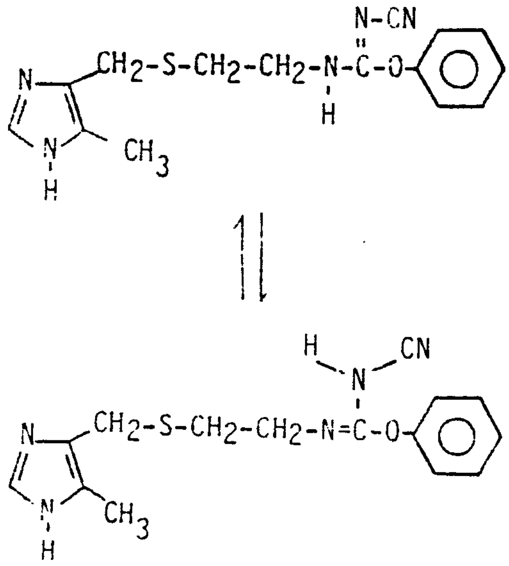

(IV)

Europäisches Patentamt

# EUROPÄISCHER RECHERCHENBERICHT

Nummer der Anmeldung

EP 85 11 5487

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 445 322 (SOGO)<br>--- | | C 07 D 233/64 |
| A | FR-A-2 508 447 (RICHTER GEDEON)<br>--- | | |
| A | EP-A-0 081 859 (SMITHKLINE BECKMAN)<br>----- | | |
| | | | RECHERCHIERTE SACHGEBIETE (Int. Cl.4) |
| | | | C 07 D 233/00 |

Der vorliegende Recherchenbericht wurde für alle Patentansprüche erstellt.

| Recherchenort | Abschlußdatum der Recherche | Prüfer |
|---|---|---|
| DEN HAAG | 05-08-1986 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82